# EUROPEAN PATENT APPLICATION

(11) **EP 2 113 248 A1**
(43) Date of publication of application: **04.11.2009**
(21) Application number: 08008208.4
(22) Date of filing: 29.04.2008
(51) Int. Cl.: A61K 9/48, A61K 31/403, A61K 9/16

(54) **Pharmaceutical composition comprising N-[2-(diethylamino)ethyl]-5-[(5-fluoro-1,2-dihydro-2-oxo-3H-indol-3-ylidene)methyl]-2-,4-dimethyl-1H-pyrrole-3-carboxamide**

(71) Applicant: Ratiopharm GmbH, 89079 Ulm (DE)
(72) Inventor: Pätz, Jana, 86424 Dinkelscherben (DE); Muskulus, Frank, 82194 Gröbenzell (DE)
(74) Representative: Teipel, Stephan

(57) **Abstract**

The invention relates to a pharmaceutical composition comprising N-[2-(diethylamino)ethyl]-5-[(5-fluoro-1,2-dihydro-2-oxo-3H-indol-3-ylidene)methyl]-2,4-dimethyl-1H-pyrrole-3-carboxamide and a process of preparing such composition.

## Description

The invention relates to a pharmaceutical composition comprising N-[2-(diethylamino)ethyl]-5-[(5-fluoro-1,2-dihydro-2-oxo-3H-indol-3-ylidene)methyl]-2,4-dimethyl-1H-pyrrole-3-carboxamide and a process of preparing such composition.

The compound N-[2-(diethylamino)ethyl]-5-[(5-fluoro-1,2-dihydro-2-oxo-3H-indol-3-ylidene)methyl]-2,4-dimethyl-1 H-pyrrole-3-carboxamide (herein also referred to as compound I) is a receptor tyrosine kinase inhibitor which is used to treat disorders like renal cell carcinoma (RCC) and gastrointestinal stromal tumor (GIST). Its activity relies on the inhibition of cellular signaling by targeting multiple receptor tyrosine kinases including platelet-derived growth factor receptors and vascular endothelial growth factor receptors. Since both kinds of receptors are involved in tumor angiogenesis and tumor cell proliferation, the simultaneous inhibition of these targets results in both reduced tumor vascularization and cancer cell death. These effects are responsible for the finally observed shrinkage of the renal cell carcinoma and gastrointestinal stromal tumor, respectively.

Compound I and its pharmaceutical effects on disorders like cancer are described in WO 01/060814. Further medical uses of compound I and its salts are inter alia known from WO 01/045689 and WO 03/035009.

WO 01/060814 discloses two processes of preparing compound I. According to these and other known manufacturing processes, compound I is obtained as a solid. One of the forms of compound I is its crystalline malic acid salt as described in WO 03/016305. The solid exhibits poor solubility in water affecting its oral bioavailability. Moreover, it has been reported that it causes manufacturing problems when being processed by higher concentrations, especially concentrations over 40 wt.% (see for example WO 04/024127).

Typically, compound I is administered in a dose of 50 mg once daily, which, if necessary, has to be varied according to individual tolerance and safety. Thus, in order to obtain sufficiently flexible dosing, individual dosage forms generally contain 12.5, 25 and 50 mg of compound I. Capsules comprising the malate salt of compound I are sold under the brand name Sutent® (by Pfizer Pharma).

The above-mentioned manufacturing problems might be attributed to the crystalline form of compound I. Moreover, good homogeneity and flowability of a pharmaceutical composition are a prerequisite for a successful manufacture of for example tablets and capsules on a production scale.

It is therefore an object of the invention to provide a pharmaceutical composition comprising compound I or a pharmaceutically acceptable salt thereof which does not encounter the above problems. In particular the composition should possess improved properties like solubility, homogeneity and flowability.

It has now been found that the above problems can be overcome by providing a certain pharmaceutical composition comprising compound I or a salt of it in amorphous form.

Thus, the present invention relates to a pharmaceutical composition comprising N-[2-(diethylamino)ethyl]-5-[(5-fluoro-1,2-dihydro-2-oxo-3H-indol-3-ylidene)methyl]-2,4-dimethyl-1 H-pyrrole-3-carboxamide or a pharmaceutically acceptable salt thereof as active pharmaceutical ingredient, wherein the active pharmaceutical ingredient is present in amorphous or partially amorphous form, characterized in that the active pharmaceutical ingredient is layered onto particles or pellets comprising one or more pharmaceutically acceptable excipients or coated with one or more pharmaceutically acceptable excipients or dispersed or dissolved in a solid melt comprising one or more pharmaceutically acceptable excipients.

N-[2-(diethylamino)ethyl]-5-[(5-fluoro-1,2-dihydro-2-oxo-3H-indol-3-ylidene)methyl]-2,4-dimethyl-1 H-pyrrole-3-carboxamide (compound I) has the following chemical structure:

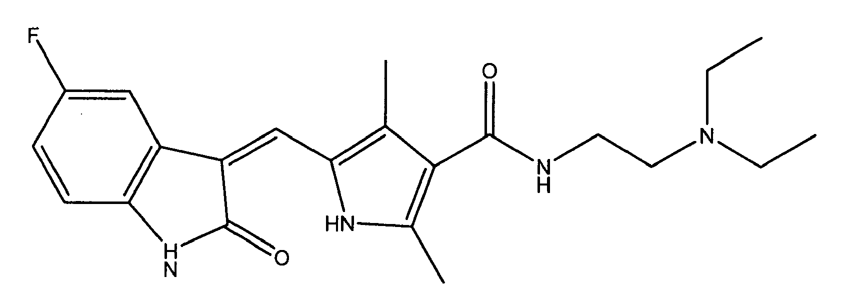

Compound I as well as its salts can be readily synthesized using techniques well known in the art. Syntheses of compound I are disclosed for example in WO 01/060814.

The term "amorphous form" refers to a form of active pharmaceutical ingredient which has no long-range order like crystalline forms. The atoms of a material present in amorphous form exist in a non-uniform array. It is for example possible to distinguish amorphous from crystalline forms of a compound by powder X-ray diffraction.

The term "active pharmaceutical ingredient" (API) refers to compound I in its salt free or salt form. Thus, if a pharmaceutically acceptable salt of compound I is employed, active pharmaceutical ingredient refers to compound I including the salt component.

The term "partially amorphous form" refers to a form of the active pharmaceutical ingredient comprising aside from a portion of it in amorphous form a portion in crystalline form.

Preferably, at least 10 wt.%, more preferably at least 20 wt.%, at least 30 wt.%, at least 40 wt.%, at least 50 wt.% of the active pharmaceutical ingredient are in amorphous form, even more preferably at least 90 wt.%, most preferably at least 98 wt.%, wherein the respective amounts being referred to the total weight of the active pharmaceutical ingredient in the composition of the present invention.

The term "pharmaceutical composition" refers to single dosage forms, such as tablets, capsules, pellets, etc., as well as powders or granules which are used in the preparation of single dosage forms. Where it is referred to the total weight of the pharmaceutical composition and the pharmaceutical composition in a single dosage form the total weight is the weight of the single dosage form excluding, if applicable, the weight of any coating or capsule shell.

The pharmaceutical composition of the present invention is characterized in that the active pharmaceutical ingredient is a) layered onto particles or pallets comprising one or more pharmaceutically acceptable excipients, or b) coated with one or more pharmaceutically acceptable excipients, or c) dispersed or dissolved in a solid melt comprising one or more pharmaceutically acceptable excipients. By this layering, coating, dispersing or dissolving the active pharmaceutical ingredient in its amorphous of partially amorphous form is finally divided and/or stabilized (chemically and/or in its amorphous form), thereby providing a composition being particularly stable and soluble.

If the active pharmaceutical ingredient is layered onto particles or pellets, the composition can be obtained for example by dispersing the particles or pellets in a solvent wherein they are non-soluble, dissolving the active pharmaceutical ingredient in this dispersion and removing the solvent. Alternatively, the particles or pellets can be dispersed in a solution of the active pharmaceutical ingredient in a suitable solvent before removal of the solvent. In a further alternative embodiment the particles or pellets may be sprayed with a solution of the active pharmaceutical ingredient and subsequently dried. Preferably, the composition wherein the active pharmaceutical ingredient is layered onto particles or pellets is obtainable by spraying the particles or pellets with a solution of the active pharmaceutical ingredient in a fluid bed dryer.

As a solvent any suitable solvent may be employed. For example, water or ethanol are suitable solvents, preferably water.

In a further embodiment the active pharmaceutical ingredient may be layered onto the particles of pellets together with at least one further pharmaceutically acceptable excipient, such as a binder. Suitable excipients are, for example, sugar, alcohols, polyvinylpyrrolidone (PVP), hydroxypropylmethyl cellulose (HPMC), and dextrines.

As the particles or pellets any suitable particle or pellet can be used, such as commercially available inert pellets or granules. Alternatively, the particles or pellets can, for example, be prepared by granulation, such as wet granulation of a mixture of a soluble and an insoluble excipient, such as mannitol and microcrystalline cellulose. Single substances can also be utilized.

Where the pharmaceutical composition of the present invention comprises the active pharmaceutical ingredient coated with one or more pharmaceutical excipients, any suitable excipient for coating the active pharmaceutical ingredient may be employed. Suitable excipients are, for example, hydroxypropylmethyl cellulose (HPMC) and polyvinylpyrrolidone (PVP). The coating process results in an at least partial amorphization of the active pharmaceutical ingredient, thereby enhancing its solubility.

The coating of the active pharmaceutical ingredient with the excipient can be conducted by any process known to a person skilled in the art. For example, the active pharmaceutical ingredient can be dispersed in a solvent in which it is not soluble and the excipient can be dissolved in this dispersion before removal of the solvent. Alternatively, the active pharmaceutical ingredient can be dispersed in a solution of the excipient in a suitable solvent before removal of the solvent. In a further embodiment a solution of the excipient may be sprayed onto particles of the active pharmaceutical ingredient, for example in a fluid bed dryer.

In the third embodiment the active pharmaceutical ingredient is dispersed or dissolved in a melt of one or more pharmaceutically acceptable excipients. When the melt is solidified, for example, by cooling, the active pharmaceutical ingredient is molecularly dispersed within the excipient(s) or converted into its amorphous or at least partially amorphous state. Any excipient suitable for the preparation of such melts can be employed. Suitable excipients for this embodiment are, for example, polyethylene glycols (PEGs), polyvinylpyrrolidone (PVP), polyvinylacetat (PVA), cellulose derivatives, sugar alcohols, methacrylates and mixtures of mono-, di- and triglycerides. Also mixtures of one or more of these excipients can be used.

Further preferred embodiments of the composition of the present invention are explained below in the description of the processes for the preparation of the composition.

The active pharmaceutical ingredient is preferably present in the pharmaceutical composition in an amount of more than 40 wt.%, more preferably at least 53 wt.% and even more preferably at least 70 wt.%, wherein the respective amounts being referred to the weight of the total composition. Further preferred portions of the active pharmaceutical ingredient in the pharmaceutical composition are more than 40 wt.%, more than 41 wt.%, more than 42 wt.%, more than 43 wt.%, more than 44 wt.%, more than 45 wt.%, more than 50 wt.%, more than 53 wt.%, more than 60 wt.% or more than 70 wt.%, wherein the respective amounts are being referred to the weight of the total composition.

Due to the amorphous form of the active pharmaceutical ingredient, the pharmaceutical composition of the present invention possesses excellent solubility, homogeneity and flowability. Further, it shows a great workability even if the portion of active pharmaceutical ingredient in the composition is higher than 40 wt.%.

Even more advantageous properties regarding solubility, homogeneity and flowability can be achieved if the pharmaceutical composition of the present invention has a mean particle size of 1 to 1500 µm, preferably 3 to 500 µm.

A bulk density of the pharmaceutical composition ranging from of 0.3 to 0.85 g/ml, preferably of 0.4 to 0.8 g/ml, more preferably of 0.4 to 0.7 g/ml is advantageous.

The pharmaceutical composition of the invention preferably possesses Hausner ratios in the range of 1.05 to 1.45, preferably of 1.1 to 1.4. The Hausner factor is the ratio of bulk density to tapped density.

The pharmaceutical composition of the present invention may further comprise one or more additional pharmaceutically acceptable excipients, such as fillers, binding agents, lubricants, glidants, antisticking agents, crystallization inhibitors and disintegrating agents. As pharmaceutically acceptable excipients conventional excipients known to the person skilled in the art may be used. See for example "Lexikon der Hilfsstoffe für Pharmazie, Kosmetik und angrenzende Gebiete", edited by H. P. Fiedler, 4th Edition, Edito Cantor, Aulendorf and earlier editions, and "Handbook of Pharmaceutical Excipients", Third Edition, edited by Arthur H. Kibbe, American Pharmaceutical Association, Washington, USA, and Pharmaceutical Press, London.

Preferred examples of the fillers are lactose, mannitol, sorbitol or microcrystalline cellulose. The filler is suitably present in an amount of 0 to 80 wt.%, preferably of 10 to 20 wt.% of the total weight of the composition.

The binding agent can for example be microcrystalline cellulose (MCC) or hydroxypropylmethyl cellulose (HPMC). Preferably the binding agent is present in an amount of 1 to 25 wt.%, more preferably at 2 to 10 wt.% of the total weight of the composition.

The lubricant is preferably a stearate, more preferably an earth alkali metal stearate, such as magnesium stearate. The lubricant is suitably present in an amount of 0.1 to 2 wt.%, preferably about 1 wt.% of the total weight of the composition.

Preferred crystallization inhibitors may be selected from the group consisting of polyvinylpyrrolidone (PVP); organic acids, e.g. citric acid; inorganic salts, e.g. ammonium carbonate; and methacrylates. The crystallization inhibitor is suitably present in an amount of 0.1 to 10 wt.%, preferably 2 to 5 wt.% of the total weight of the composition.

Preferred disintegrating agents are croscarmellose sodium, sodium carboxymethyl starch or cross-linked polyvinylpyrrolidone (crospovidone). The disintegrating agent is suitably present in an amount of 0.1 to 20 wt.%, more preferably at about 0.5 to 7 wt.% of the total weight of the composition.

The glidant can for example be colloidal silicon dioxide. Preferably the binding agent is present in an amount of 0.5 to 8 wt.%, more preferably at 0.5 to 3 wt.% of the total weight of the composition.

The antisticking agent is for example talcum and may be present in amounts of 1 to 5 %.wt, more preferably in an amount of 1.5 to 3 wt.% of the total weight of the composition.

A person skilled in the art may use these or other excipients in regard to the selected process of preparing the pharmaceutical composition of the invention.

The pharmaceutical composition of the present invention can be formulated in any known matter, preferably as tablets, capsules, granules, pellets or sachets. A particularly preferred pharmaceutical composition is in the form of capsules. The pharmaceutical composition may contain dosage amounts of 12.5, 25 and 50 mg of the active pharmaceutical ingredient. Thus the administered amount can be readily varied according to individual tolerance and safety warranting more flexible dosing than the standard dose of 50 mg once daily.

A further aspect of the present invention provides a process of preparing a pharmaceutical composition comprising compound I or a pharmaceutically acceptable salt thereof as active pharmaceutical ingredient wherein the active pharmaceutical ingredient is present in amorphous or partially amorphous form. The process comprises the following steps:
(a) providing N-[2-(diethylamino)ethyl]-5-[(5-fluoro-1,2-dihydro-2-oxo-3H-indol-3-ylidene)methyl]-2,4-dimethyl-1H-pyrrole-3-carboxamide or a pharmaceutically acceptable salt thereof as active pharmaceutical ingredient and at least one pharmaceutically acceptable excipient; and
(b) transferring the active pharmaceutical ingredient into its amorphous or partially amorphous form by layering the active pharmaceutical ingredient onto particles or pellets comprising one or more pharmaceutically acceptable excipients or by coating the active pharmaceutical ingredient with one or more pharmaceutically acceptable excipients or by dispersing or dissolving the active pharmaceutical ingredient in a melt comprising one or more pharmaceutically acceptable excipients.

In the first embodiment of the process of the present invention the active pharmaceutical ingredient is layered onto particles or pellets comprising one or more excipients, leading to amorphization or partial amorphization of the active pharmaceutical ingredient.

Preferably, for the formation of said particles or pellets a mixture of a soluble and an insoluble excipient such as mannitol and microcrystalline cellulose is used. Single substances can also be utilized.

The particles or pellets can, for example, be sprayed with an aqueous solution of the active pharmaceutical ingredient additionally containing one or more of the following excipients: sugar alcohols, PVP, HPMC and dextrins. Depending on the excipients and the granulation parameters, mean particle sizes of 20 to 1500 µm and bulk densities of 0.5 to 0.85 g/ml can be obtained. The granulates according to this embodiment of the present invention can have Hausner ratios between 1.05 and 1.35.

In the second embodiment of the process of the present invention the active pharmaceutical ingredient is coated with one or more excipients such as HPMC or PVP that enhance the solubility of the active pharmaceutical ingredient. This coating process leads to an at least partial amorphization of the active pharmaceutical ingredient. Depending on the particle size of the active pharmaceutical ingredient and the process parameters mean particle sizes of 3 to 500 µm and bulk densities between 0.3 to 0.8 g/ml can be obtained. The granulates according to this embodiment of the present invention can have Hausner ratios between 1.1 and 1.45.

In the third embodiment of the process of the present invention the active pharmaceutical ingredient is dispersed or dissolved in a melt of one or more excipients. After the melt has been cooled, the active pharmaceutical ingredient is molecularly dispersed within the excipient(s) or passes into an amorphous or partially amorphous state. Preferably, excipients such as polyethylene glycols (PEGs), PVP, polyvinyl acetate (PVA), cellulose derivatives, sugar alcohols, methacrylates and mixtures of mono- , di- and triglycerides are employed. Depending on the excipients and the milling technology chosen, mean particle sizes of 1 to 500 µm and bulk densities of 0.4 to 0.8 g/ml can be obtained. Granulates according to this embodiment of the present invention can have Hausner ratios between 1.05 and 1.4.

The invention is now illustrated in the following examples which are not to be construed to be limiting.

### Examples

**Example 1:**

| **Ingredient** | **Product** | **Function** | **25 mg dosage** |
|---|---|---|---|
| Compound I | | API | 25 |
| HPMC | Klucel | Binder | 3.0 |
| Lactose | Flow lac | filler | 15.0 |
| Sodium croscarmellose | Ac-Di-SOL | disintegrant | 3.0 |
| Magnesium stearate | tbd | antisticking | 0.6 |
| **Total** | | | 46.6 |

The API particles (mean particle size 0.4 to 400 µm) are sprayed with an aqueous solution of 5 to 20 % of HPMC in a fluid bed dryer (nozzle width 0.8 to 1.2 mm, inlet temperature 50 to 70 °C, product temperature about 40 °C) and afterwards dried for up to 1.5 hours at 30 to 50 °C. Lactose and sodium croscarmellose are mixed with the coated granules. Finally, magnesium stearate is added. The mixture is filled into hard gelatine capsules (size 4).

**Example 2a:**

| **Ingredient** | **Product** | **Function** | **25 mg dosage** |
|---|---|---|---|
| Compound I | | API | 25 |
| MCC | Anvicel PH101 | filler | 12.8 |
| PVP | Kollidon 25 | binder | 5.0 |
| Sodium croscarmellose | Ac-Di-SOL | disintegrant | 3.0 |
| Colloidal silicon dioxide | Aerosil | glidant | 0.2 |
| Magnesium stearate | tbd | antisticking | 0.6 |
| **Total** | | | 46.6 |

MCC, 0.1 mg colloidal silicon dioxide and 0.3 mg magnesium stearate are fluid bed granulated with a suspension of API and PVP in water (5 to 50 %). The resulting granules are sieved (mesh width 0.5 to 1.2 mm). Then, sodium croscarmellose and the residual colloidal silicon dioxide and magnesium stearate are added and mixed with the granules. The mixture is filled into hard gelatine capsules (size 4).

**Example 2b:**

| **Ingredient** | **Product** | **Function** | **25 mg dosage** |
|---|---|---|---|
| Compound I | | API | 25 |
| Pellets | Neutralpellets | | 50 |
| HPMC | Pharmacoat 603 | binder | 15 |
| Talcum | Talcum | antisticking | 0.3 |
| **Total** | | | 90.3 |

Alternatively, commercially available pellets are sprayed with an aqueous solution of API, HPMC and talcum at a temperature of 30 to 50 °C. Afterwards, the pellets are dried for up to 1.5 hours at 30 to 45 °C. The coated pellets are filled into hard gelatine capsules (size 4).

**Example 3:**

| **Ingredient** | **Product** | **Function** | **25 mg dosage** |
|---|---|---|---|
| Compound I | | API | 25 |
| Sorbitol | Neosorb | filler | 18.4 |
| Crospovidone | Kollidon CL | disintegrant | 2.5 |
| Colloidal silicon dioxide | Aerosil | glidant | 0.2 |
| Magnesium stearate | tbd | antisticking | 0.5 |
| **Total** | | | 46.6 |

API and sorbitol are melt extruded in a twice screw extruder at 110 to 180 °C. The extrudat is sieved (mesh width 0.5 to 1.2 mm) or spheronized and mixed with crospovidone, colloidal silicon dioxide and magnesium stearate. The mixture is filled into hard gelatine capsules (size 4).

## Claims

1. A pharmaceutical composition comprising N-[2-(diethylamino)ethyl]-5-[(5-fluoro-1,2-dihydro-2-oxo-3H-indol-3-ylidene)methyl]-2,4-dimethyl-1H-pyrrole-3-carboxamide or a pharmaceutically acceptable salt thereof as active pharmaceutical ingredient, wherein the active pharmaceutical ingredient is present in amorphous or partially amorphous form, **characterized in that** the active pharmaceutical ingredient is layered onto particles or pellets comprising one or more pharmaceutically acceptable excipients or coated with one or more pharmaceutically acceptable excipients or dispersed or dissolved in a solid melt comprising one or more pharmaceutically acceptable excipients.

2. The pharmaceutical composition according to claim 1, comprising the active pharmaceutical ingredient in an amount of more than 40 % by weight of the total weight of the composition.

3. The pharmaceutical composition according to any of the preceding claims, having a mean particle size of 1 to 1500 µm.

4. The pharmaceutical composition according to any of the preceding claims, having a bulk density of 0.3 to 0.85 g/ml.

5. The pharmaceutical composition according to any of the preceding claims, having a Hausner ratio of 1.05 to 1.45.

6. The pharmaceutical composition according to any of the preceding claims, further comprising one or more additional pharmaceutically acceptable excipients, such as fillers, binding agents, lubricants, glidants, crystallization inhibitors and disintegrating agents.

7. A process of preparing a pharmaceutical composition according to any of claims 1 to 6, comprising
(a) providing N-[2-(diethylamino)ethyl]-5-[(5-fluoro-1,2-dihydro-2-oxo-3H-indol-3-ylidene)methyl]-2,4-dimethyl-1 H-pyrrole-3-carboxamide or a pharmaceutically acceptable salt thereof as active pharmaceutical ingredient and at least one pharmaceutically acceptable excipient; and
(b) transferring the active pharmaceutical ingredient into its amorphous or partially amorphous form by layering the active pharmaceutical ingredient onto particles or pellets comprising one or more pharmaceutically acceptable excipients or by coating the active pharmaceutical ingredient with one or more pharmaceutically acceptable excipients or by dispersing or dissolving the active pharmaceutical ingredient in a melt comprising one or more pharmaceutically acceptable excipients.

8. The process according to claim 7, wherein the active pharmaceutical ingredient is layered onto particles or pellets comprising a mixture of a soluble and an insoluble excipient.

9. The process according to claim 8, wherein said soluble excipient is mannitol and said insoluble excipient is microcrystalline cellulose.

10. The process according to claim 7, wherein the active pharmaceutical ingredient is coated with one or more solubility enhancing excipients.

11. The process according to claim 10, wherein the solubility enhancing excipient is selected from hydroxypropylmethyl cellulose, polyvinylpyrrolidone and mixtures thereof.

12. The process according to claim 7, wherein the active pharmaceutical ingredient is dispersed or dissolved in a melt of one or more excipients selected from the group consisting of polyethylene glycols, polyvinylpyrrolidone, polyvinyl acetate, cellulose derivatives, sugar alcohols, methacrylates, mixtures of mono-, di - and triglycerides and mixtures of these exipients.

13. A pharmaceutical composition obtainable by the process according to any of claims 7-12.
